# EUROPEAN PATENT SPECIFICATION

(11) **EP 4 324 497 B1**
(45) Date of publication and mention of the grant of the patent: **18.11.2026**
(21) Application number: 23192073.7
(22) Date of filing: 18.08.2023
(51) Int. Cl.: A61M 5/142, A61B 5/00, A61F 13/0246, A61K 9/00, A61M 5/158, A61M 25/02

(54) **ADHESIVE LAYER WITH MULTIPLE STRENGTHS OF ADHESIVES FOR SECURING AN ON-BODY MEDICAL DEVICE TO A USER**
HAFTSCHICHT MIT MEHREREN HAFTSTÄRKEN ZUR BEFESTIGUNG EINER AM KÖRPER TRAGBAREN MEDIZINISCHEN VORRICHTUNG AN EINEM BENUTZER
COUCHE ADHÉSIVE À FORCES MULTIPLES D'ADHÉSIFS POUR FIXER UN DISPOSITIF MÉDICAL SUR LE CORPS À UN UTILISATEUR

(30) Priority: 18.08.2022 US 202263371776 P
(43) Date of publication of application: 21.02.2024
(73) Proprietor: Insulet Corporation, Acton, MA 01720 (US)
(72) Inventor: BREINGAN, Kyle, Lowell (US); DOUDOUMOPOULOS, Alexander, Boxborough (US)
(74) Representative: Peterreins Schley

(56) References cited:
- WO-A1-2018/216652
- US-A1- 2009 198 215
- US-A1- 2011 022 002
- US-A1- 2016 279 325
- US-A1- 2021 161 720
- US-B1- 6 491 995

## Description

### BACKGROUND

On-body medical devices often include adhesive layers for securing the on-body medical devices to the user. The adhesive layers contain adhesives that adhere to the skin of the users to hold the on-body medical devices in place on the users. To secure such an on-body medical device to the skin, the user presses the adhesive layer against the skin until the adhesive layer securely adheres to the skin. Most on-body medical devices have fairly short lifespans of use, such as for a few hours or a few days. When the end of the lifespan of use is reached, the on-body medical device is removed by peeling the adhesive layer off of the skin of the user.

Choosing the best adhesive for an adhesive layer of an on-body medical device can be difficult. The adhesive must be strong enough to keep the on-body medical device secured to the user for the entire lifespan of use. Otherwise, the adhesive may fail and the on-body medical device may become detached from the user. However, the adhesive also must not be too strong. If the adhesive is too strong, it may be painful for the user to peel off the adhesive layer at the end of the lifespan of use. A too strong adhesive also may cause skin damage to the user when the adhesive layer is peeled off. Most conventional on-body medical devices tend to have strong adhesives to prevent detachment of the on-body medical devices from the skins of the users. Some prior art on-body medical devices comprise adhesive layers with more than one adhesive, the adhesives having different adhesive strengths. Examples of such devices are known from e.g. US 2011/022002 A1 or US 2009/198215 A1.

### SUMMARY

The invention comprises an on-body medical device according to claim 1, and a method of securing an adhesive layer to an on-body device as defined in claim 11. Additional features of preferred embodiments of the invention are specified in the respective dependent claims. In accordance with a facet, an on-body medical device includes a skin-facing surface configured for facing the skin of a user. The device also includes an adhesive layer secured to the skin-facing surface for securing the on-body medical device to the skin of the user. The adhesive layer includes a stronger adhesive positioned around an outer perimeter of the adhesive layer and a weaker adhesive positioned inside the outer perimeter of the adhesive layer. The stronger adhesive is at least 20% stronger than the weaker adhesive.

The on-body medical device may additionally include a needle and/or cannula insertion assembly for inserting a needle and/or cannula through the skin of the user to deliver a medicament to the user. The device may also include an opening in the skin-facing surface, and an opening in the adhesive layer that is aligned with the opening in the skin facing surface to create a path for the needle and/or cannula to pass for enabling the needle and/or the cannula to be inserted through the skin of the user. The stronger adhesive also may surround a perimeter of the opening in the adhesive layer. The device may include an intermediate strength adhesive that surrounds the opening in the adhesive layer. The intermediate strength adhesive may be weaker than the stronger adhesive but stronger than the weaker adhesive. The weaker adhesive may be a 1 day wear time adhesive or a 2 day wear time adhesive. The stronger adhesive may be a 3 day wear time adhesive or a 4 day wear time adhesive. In this case, "wear time" may refer to an average wear time for a typical user under typical conditions. Alternatively, the stronger adhesive may be 10% to 100% stronger than the weaker adhesive, or more particularly, 10% to 50%; or in an exemplary embodiment, 20% stronger. The on-body medical device may be a medicament delivery device. The medicament delivery device may be an insulin pump. The skin-facing surface may be a surface on the housing of the on-body medical device. The device may include a hole in the adhesive layer, and the stronger adhesive may be positioned on the adhesive layer around a perimeter of the hole.

In accordance with another facet, an on-body medical device includes a housing having a skin-facing surface configured for facing the skin of a user. The device also includes an adhesive layer secured to the skin-facing surface of the housing for securing the on-body medical device to the skin of the user. The adhesive layer includes what may be termed "islands" on the adhesive layer where no adhesive is present or where a weaker strength adhesive (relative to the other adhesive) is present, and may include a stronger strength adhesive applied around a perimeter of the adhesive layer, and may also include a stronger strength adhesive around a region interior to the perimeter so as to surround the islands. Accordingly, the islands of no adhesive or weaker strength may be defined by their complete perimeter being surrounded by a stronger adhesive. The stronger strength adhesive may be 10% to 100% stronger than the weaker adhesive, or more particularly, 10% to 50%; or in an exemplary embodiment, at least 20% stronger than the weaker strength adhesive.

The islands may be periodically spaced apart. The islands may be arranged in a grid. The islands may have the weaker adhesive present, and the weaker adhesive may be a 1 day wear time or a 2 day wear time adhesive. The stronger adhesive may be a 3 day wear time or a 4 day wear time adhesive. In some embodiments, the stronger adhesive may be a 3 to 28 days wear time adhesive, more specifically a 3 to 10 days wear time adhesive and in particular a 3 to 5 days wear time adhesive. A 1 day wear time adhesive may be defined as an adhesive that loses more than 50% of its peel strength within 1 day of being in contact with the user's skin. A 3 day wear time adhesive may be defined as an adhesive that loses more than 50% of its peel strength within 3 day of being in contact with the user's skin, but less than 50% within 2 days of being in contact with the user's skin. In some embodiments, the stronger adhesive is an acrylate based adhesive. In some embodiments, the weaker adhesive is a silicone based adhesive.

In accordance with an additional facet, an on-body medical device includes a skin-facing surface configured for facing the skin of a user and an adhesive layer secured to the skin-facing surface for securing the on-body medical device to the skin of the user. In reverse of the earlier embodiment, the adhesive layer may include "islands" on the adhesive layer where a stronger strength adhesive is present and a weaker strength adhesive applied around a perimeter of the adhesive layer and also applied around a region interior to the perimeter so as to surround the islands with the weaker strength adhesive. Accordingly, the islands of stronger adhesive may be defined by their complete perimeter being surrounded by a weaker adhesive. The stronger strength adhesive may be 10% to 100% stronger than the weaker adhesive, or more particularly, 10% to 50%; or in an exemplary embodiment, at least 20% stronger than the weaker strength adhesive.

The islands may be periodically spaced apart. The islands may be arranged in a grid. The device may further include a needle and/or cannula insertion assembly for inserting a needle and/or cannula through the skin of the user to deliver a medicament to the user, an opening in the skin-facing surface, and a corresponding opening in the adhesive layer for enabling the needle and/or the cannula to be inserted through the skin of the user.

In another exemplary embodiment, the adhesive may form a gradient of strength from an outer perimeter (where the adhesive may be stronger) toward the center (where the adhesive may be weaker). The stronger adhesive at the perimeter may be 10% to 100% stronger than the weaker adhesive, or more particularly, 10% to 50%; or in an exemplary embodiment, 20% stronger. And moving in from the perimeter, the adhesive may gradually weaken until it becomes what may be termed the "weaker adhesive." This gradient may be created by applying different amounts of adhesive across the surface of the adhesive layer, such that the perimeter has a greater amount of adhesive material than the interior or near the center of the adhesive layer, which may result in a stronger adhesive property.

In a further exemplary embodiment, a portion of the perimeter may have a weaker adhesive then other portions of the perimeter or than the rest of the perimeter. For example, a bottom end of the adhesive layer may have a weaker adhesive that is easier to separate from a user's skin, so as to allow the user to insert their finger at that portion under the adhesive layer and above the skin when it comes time to remove the adhesive layer from the skin. This portion may be arcuate in shape or in the shape of a fingertip. By allowing a user to insert their fingertip at this weaker adhesive portion when it comes time to remove the adhesive layer from the skin, it may be easier for the user to start the adhesive removal process of the rest of the adhesive layer.

### BRIEF DESCRIPTION OF THE DRAWINGS

Figure 1A depicts a side view of an illustrative on-body medical device of an exemplary embodiment that includes an adhesive pad.
Figure 1B depicts a partially exploded view of the adhesive pad and the housing of the on-body medical device of Figure 1A.
Figure 2 depicts a flowchart of illustrative steps that may be performed in exemplary embodiments to create and secure an adhesive layer to an on-body medical device.
Figure 3 depicts an illustrative bottom surface of an on-body medical device of an exemplary embodiment having at least two adhesives of different strengths on an adhesive layer.
Figure 4 depicts a flowchart of illustrative steps that may be performed in exemplary embodiments to create an adhesive layer having at least two adhesives of different strengths.
Figure 5 depicts an illustrative bottom surface of an on-body medical device of an exemplary embodiment having adhesives of at least three strengths on an adhesive layer.
Figure 6 depicts a flowchart of illustrative steps that may be performed in exemplary embodiments to create an adhesive layer having at least three adhesives of different strengths.
Figure 7 depicts an illustrative bottom surface of an on-body medical device of an exemplary embodiment having "islands" of adhesive or lack of adhesive and a surrounding region.
Figure 8 depicts a flowchart of illustrative steps that may be performed in exemplary embodiments to apply a stronger adhesive to islands and a weaker adhesive to surrounding spaces on an adhesive layer of an on-body medical device.
Figure 9A depicts an illustrative bottom surface of an on-body medical device of an exemplary embodiment where holes and other structures may have a stronger adhesive applied around the holes or structures to prevent peeling.
Figure 9B depicts an illustrative bottom surface of an on-body medical device of an exemplary embodiment where adhesive forms a gradient of lessening strength as one moves radially inward from a stronger adhesive at the perimeter to a weaker adhesive at the center of the on-body medical device.
Figure 9C depicts an illustrative bottom surface of an on-body medical device of an exemplary embodiment where a portion of the perimeter has a weaker adhesive then other portions of the perimeter or than the rest of the perimeter to allow the user to insert their finger at that portion under the adhesive layer.

### DETAILED DESCRIPTION

In exemplary embodiments, an on-body medical device has an adhesive layer with adhesives of two or more strengths. In some of the exemplary embodiments, a stronger adhesive is positioned on the adhesive layer where additional strength is needed, such as around the perimeter of the adhesive layer to prevent premature peeling. A weaker adhesive is positioned on other portions of the adhesive layer where there is no need for the strength of the stronger adhesive. For example, the interior portion of the adhesive layer inside the perimeter may be coated with the weaker adhesive. The use of the weaker adhesive in the interior may reduce the discomfort to a user when peeling off the adhesive layer and may decrease the risk of skin damage. The stronger adhesive around the perimeter may still securely hold the adhesive layer to the skin of the user but may occupy only a thin band around the perimeter. As a result, it may not be as painful to peel off the adhesive layer of exemplary embodiments as it is to peel off a conventional adhesive layer with a strong adhesive coated on the entire adhesive layer since the band of stronger adhesive used in the exemplary embodiments only occupies a small surface area.

In other exemplary embodiments, at least three strengths of adhesives are used on the adhesive layer. An intermediate strength adhesive is used along with a stronger adhesive and a weaker adhesive. The stronger adhesive and weaker adhesive may be positioned largely as discussed above, but the intermediate strength adhesive may be used in areas where the weaker adhesive does not provide enough strength but the full strength of the stronger adhesive is not needed.

In some exemplary embodiments, the adhesives may be applied continuously over designated regions that cover large portions of the surface area of the adhesive layer. In other exemplary embodiments, one or more of the adhesives may be applied in small discrete regions, such as dots or islands, which may be surrounded by other adhesives on the adhesive layer. The discrete adhesive regions may be organized into a pattern, such as a grid. The discrete regions may provide discrete points or islands of stronger adhesive or discrete points or islands of weaker strength adhesive in exemplary embodiments.

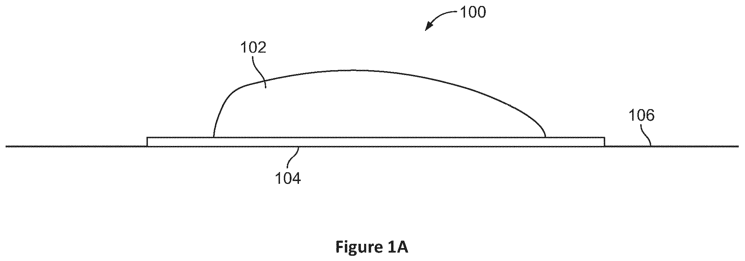

Figure 1A depicts an illustrative on-body medical device 100 for an exemplary embodiment. The on-body medical device 100 may be, for example, a monitor or a sensor. For example, the on-body medical device 100 may be a heart rate monitor, a blood pressure monitor, a glucose sensor, a ketone sensor, or the like. The on-body medical device 100 may be a medicament delivery device. The medicament delivered by the on-body medical device 100 may be insulin, pramlintide, GLP-1 or a co-formulation of two or more of the foregoing for lowering a user's glucose level, or may be glucagon for raising a user's glucose level. The medicament may be a glucagon-like peptide (GLP)-1 receptor agonist for lowering glucose or slowing gastric emptying, thereby delaying spikes in glucose after a meal. Alternatively, the medicament delivered by the medicament delivery device 102 may be one of a pain relief agent, a chemotherapy agent, an antibiotic, a blood thinning agent, a hormone, a blood pressure lowering agent, an antidepressant, an antipsychotic, a statin, an anticoagulant, an anticonvulsant, an antihistamine, an anti-inflammatory, a steroid, an immunosuppressive agent, an antianxiety agent, an antiviral agent, a nutritional supplement, a vitamin, or co-formulations of two or more of the above.

More generally, the on-body medical device 100 is a device that is adhered to the user and serves a medical purpose. The on-body medical device 100 includes at least one housing 102 for housing components of the on-body medical device. The at least one housing may be positioned on or in a tray, which may previously have been attached to the skin of the user, and the tray may be attached to the adhesive layer. Alternatively, the at least one housing 102 may be attached to the adhesive layer as described in exemplary embodiments below. The at least one housing 102, may be made of a material, such as a plastic, a composite, a metal or a combination thereof. For example, suppose that the on-body medical device 100 is an insulin pump. In that case, the at least one housing 102 may house the mechanical and electrical components of the insulin pump, like an insulin reservoir, a microprocessor, a memory, a pump, batteries and the like. An adhesive layer 104 is secured to a surface of the on-body medical device 100. In Figure 1A, the adhesive layer 104 is secured to a bottom exterior surface of the housing by thermal weld or another securing mechanism, such as an adhesive or a mechanical fastener. In other embodiments, the adhesive layer 104 is secured to a bottom exterior surface of a tray by thermal weld or another securing mechanism, such as an adhesive or mechanical fastener, and the tray may serve as a foundation for the at least one housing 102 and the various other elements of the on-body medical device 100. The adhesive layer 104 includes multiple strengths of adhesives on it for securing the on-body medical device 100 to the skin 106 of a user. The adhesive layer 104 may be formed of a base or substrate material upon which the adhesives are coated, applied, or secured.

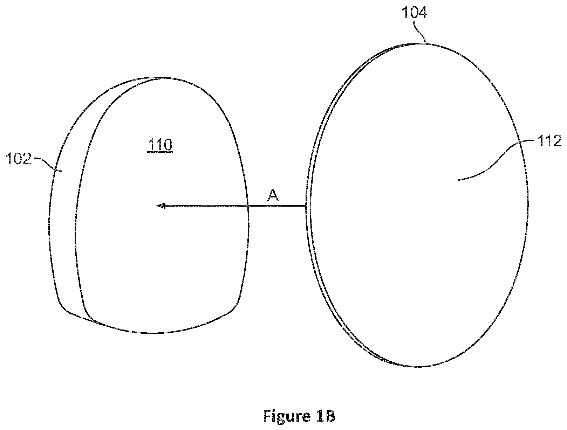

Figure 1B depicts a partially exploded view of a bottom exterior surface 110 of the housing 102 to which the adhesive layer 104 is secured as indicated by arrow A. The circumference of the outer border of the adhesive layer 104 may be larger than the circumference of the outer border of the surface 110. Surface 112, also referred to as first surface, of the adhesive layer 104 is the surface on which the adhesives are present and is the surface that is secured to the skin 106 of the user (when the on-body medical device 100 is attached to the user).

In some embodiments, the surface to which the adhesive layer 104 is secured is not part of the housing 102. Instead, the surface may be part of a stand, feet or other element that is configured to be skin-facing (i.e., oriented to face the skin of the user). Accordingly, in some embodiments, the surface is an outer surface of the on-body medical device 100. That the surface is skin-facing does not require the surface to be in direct contact with the skin of the user, when the medical device 100 is attached to the user, in particular the adhesive layer 104 may be disposed between the surface and the skin 106 of the user. Moreover, there need not be a single adhesive layer. There may be multiple adhesive layers for multiple contact areas with the skin of the user.

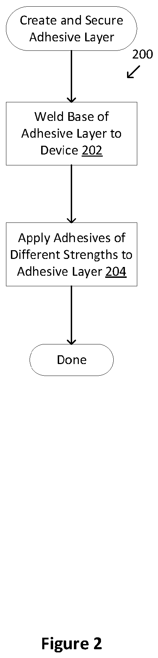

Figure 2 depicts a flowchart 200 of illustrative steps that may be performed in exemplary embodiments to create and secure the adhesive layer. The base or substrate (hereinafter "base") of the adhesive layer may be a layer of a material, such as a layer of a polyester woven material. At 202, this base of the adhesive layer is welded to a skin-facing surface of the on-body medical device. The skin-facing surface of the on-body medical device may be the first surface described above. The weld may be a thermal weld formed by melting a plastic weld structure formed on the surface. As was mentioned above, in other embodiments, the base may be secured to the surface by other means, like an adhesive or a mechanical fastener. At 204, adhesives of different strengths may be applied to the base of the adhesive layer. This application may entail coating portions of the adhesive layer with the adhesives. In some embodiments, the bottom layer of adhesive on the adhesive layer may be of greater strength and an adhesive of lower strength may be applied on top of the greater strength adhesive at selected areas. Those skilled in the art will appreciate that various techniques may be used to apply the adhesives to the adhesive layer.

Strength of the adhesives may be measured and classified in different ways. For example, the adhesives may be measured by peel strength. The peel strength may be determined for example according to ISO 8510-2:2006-12 or DIN EN 28510-1:2014-07. The peel strength may be expressed as N/mm. The adhesives strengths also may be measured and classified by how long the adhesive are intended to be adhered to the skin of a typical user experiencing typical use conditions. For example, an adhesive may be classified as a two day wear time adhesive if the adhesive is intended to securely hold for two days on the skin of a typical user with average skin properties (e.g., properties such as perspiration level at the particular skin surfaces where on-body medical devices are typically worn).

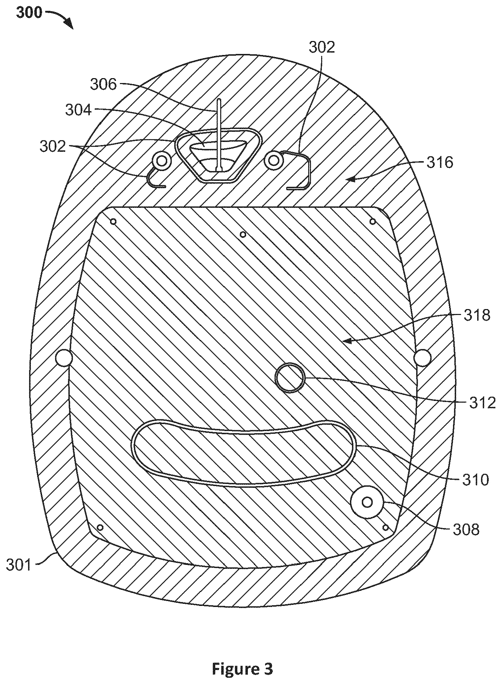

Figure 3 depicts the bottom surface of an on-body medical device 300 with an adhesive layer 301 attached. In this depicted example, the on-body medical device 300 is an insulin pump that delivers insulin to a user. The adhesive layer 301 includes adhesives of two different strengths. A stronger adhesive 316 is on the outer perimeter of the adhesive layer and around a needle/cannula assembly that includes an opening 304 through which a needle and/or cannula 306 may exit to puncture the skin of the user and through which the needle or cannula may retract back into the housing of the on-body medical device. Thermal welds 302 are formed around the needle/cannula assembly to ensure that the adhesive layer 301 does not detach from the bottom surface of the housing. The higher strength adhesive 316 is positioned around the needle/cannula assembly to ensure that the needle and/or cannula 306 does not move or shift once the adhesive layer 301 is applied to the skin of the user. A weaker adhesive 318 is on the adhesive layer 301 in the interior area inside the perimeter of the adhesive layer 301. The weaker adhesive 318 covers the area over the large weld 310 and valve 312. The weaker adhesive surrounds a needle port 308 for adding insulin to a reservoir.

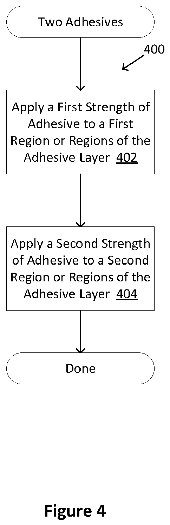

Figure 4 depicts a flowchart 400 of illustrative steps that may be performed in exemplary embodiments to create an adhesive layer with two strengths of adhesive like that shown in Figure 3. At 402, an adhesive of a first strength is applied to a first region or regions of the adhesive layer. For example, in Figure 3, the stronger adhesive 316 may be applied to the entire adhesive layer 301 or merely to the perimeter and around the needle/cannula assembly. At 404, an adhesive of a second strength is applied to a second region or regions of the adhesive layer. With respect to the example of Figure 3, the weaker adhesive 318 is applied to cover the indicated interior region either by direct application of the weaker adhesive 318 to the base of the adhesive layer 301 or on top of the stronger adhesive in the indicated interior region.

The stronger adhesive 316 may be 10% to 100% stronger than the weaker adhesive, or more particularly, 10% to 50%; or in an exemplary embodiment, 20% stronger than the weaker adhesive 318. The strength may be measured as peel strength or as wear time. "Wear time" may refer to an average wear time for a typical user (with typical skin properties) under typical use conditions. The peel strength of the stronger adhesive may be at least 20% stronger than the peel strength of the weaker adhesive 318 in an exemplary embodiment. Alternatively, the wear time of the stronger adhesive 316 may be at least 20% greater than the wear time of the weaker adhesive 318 in an exemplary embodiment. For example, the weaker adhesive may be a 1 or 2 day "wear time" adhesive, whereas the stronger adhesive may be a 3 or 4 day "wear time" adhesive, for an on-body medical device intended to be adhered to the user for 3 or 4 days. Thus, the stronger adhesive may be 50% or even 100% stronger in some instances, as suggested above.

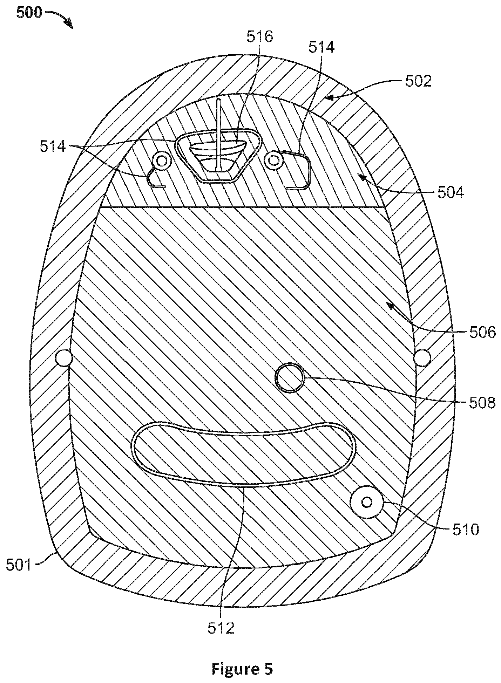

As was mentioned above, the adhesive layer also may have more than two strengths of adhesives on it. Figure 5 depicts an illustrative bottom surface of an on-body medical device 500 with an adhesive layer 501 having adhesives 502, 504 and 506 of different strengths. A stronger adhesive 502 is positioned around the perimeter of the adhesive layer 501. An intermediate strength adhesive 504 is positioned around the needle/cannula hole 516 and welds 514 to provide enhanced adhesive strength relative to the weaker adhesive 506. The weaker adhesive 506 is positioned around the interior of the adhesive layer around the region where valve 508, port 510 and weld 512 are located. This approach provides enhanced strength around the needle/cannula assembly but with an adhesive that is not as strong as the stronger adhesive. As such, the portion with the intermediate strength adhesive 514 is less painful to peel off and less likely to cause skin damage when peeled off than the same portion of the adhesive layer in the embodiment of Figure 3. The stronger adhesive 502 may be 10% to 50% stronger than the intermediate strength adhesive 504, or more particularly, 10% to 30%; or in an exemplary embodiment, approximately 15% stronger than the intermediate strength adhesive 504. Similarly, the intermediate strength adhesive 504 may be 10% to 50% stronger than the weaker adhesive 506, or more particularly, 10% to 30%; or in an exemplary embodiment, approximately 15% stronger than the weaker strength adhesive 506.

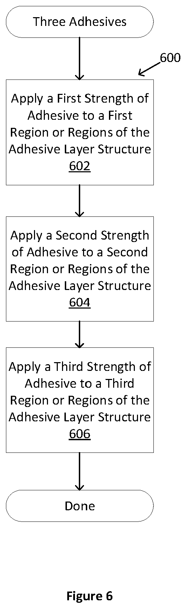

Figure 6 depicts a flowchart 600 of illustrative steps that may be performed in exemplary embodiments to create an adhesive layer with three strengths of adhesives. At 602, a first strength of adhesive may be applied to a first region or regions of the adhesive layer as part of an adhesive layer. For example, in Figure 5, the stronger adhesive 502 is applied to the perimeter of the adhesive layer 501. At 604, a second strength of adhesive may be applied to a second region or regions of the adhesive layer. For example, in Figure 5, the intermediate strength adhesive 504 is applied inside the perimeter near the needle/cannula assembly or patient interface, or portion where an element extends out of the at least one housing and/or tray and through the skin of the user. At 606, a third strength of adhesive may be applied to a third region or regions of the adhesive layer as part of an adhesive layer. In Figure 5, the third region is the region where the weaker adhesive 506 is applied.

It should be appreciated that adhesives with more than three strengths may be applied to an adhesive layer. The number of adhesive strengths used on a particular adhesive layer may depend on the needs for a variety of adhesion strengths on the adhesive layer.

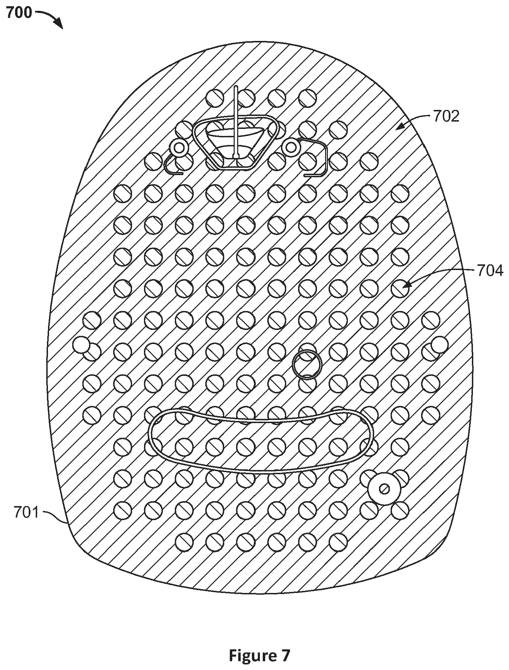

The adhesive layer need not be limited to having adhesives of different strengths applied to large continuous regions. Instead, an adhesive of a given strength may be applied to noncontinuous discrete locations on the adhesive layer. Figure 7 depicts the bottom surface 700 of an on-body medical device with an adhesive layer 701. The adhesive layer 701 has a grid of discrete points or islands (both are referred to as "islands" herein) 704 of the weaker strength adhesive. The islands 704 are surrounded, in particular the islands' 704 entire perimeter is surrounded, by a region 702 to which the stronger adhesive is applied. The region 702 encompasses the perimeter of the adhesive layer 701 and the spaces between the islands 704. This pattern of adhesives provides secure support around the perimeter of the adhesive layer 701 and adds some additional strength in the interior of the adhesive layer 701.

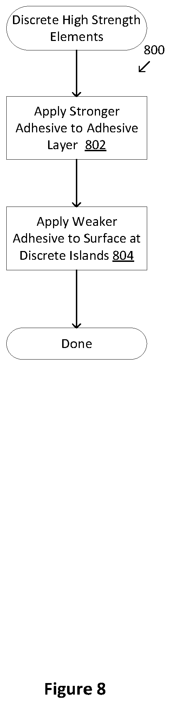

Figure 8 depicts a flowchart 800 of illustrative steps that may be performed in exemplary embodiments to create an adhesive layer with islands of adhesive like that shown in Figure 7. At 802, a stronger adhesive is applied to the adhesive layer. The stronger adhesive may be applied across the entire surface of the adhesive layer where there are no holes, ports, or the like. Alternatively, the stronger adhesive may be applied with a mask or other construct to leave the islands 704 without adhesive. At 804, the weaker adhesive is applied on top of the stronger adhesive at the islands 704 using a mask or other construct. It should be noted that in case the weaker adhesive is applied on to top of the stronger adhesive, the islands may be defined by their entire perimeter being surrounded by a stronger adhesive at the interface of the weaker adhesive and the stronger adhesive.

Alternatively, the islands 704 instead may be where the stronger adhesive is applied, and the area 702 may be where the weaker adhesive is applied or even no adhesive is applied. Moreover, in some embodiments, the islands 704 may be regions where no adhesive is applied and an intermediate strength adhesive or stronger strength adhesive is applied to region 702.

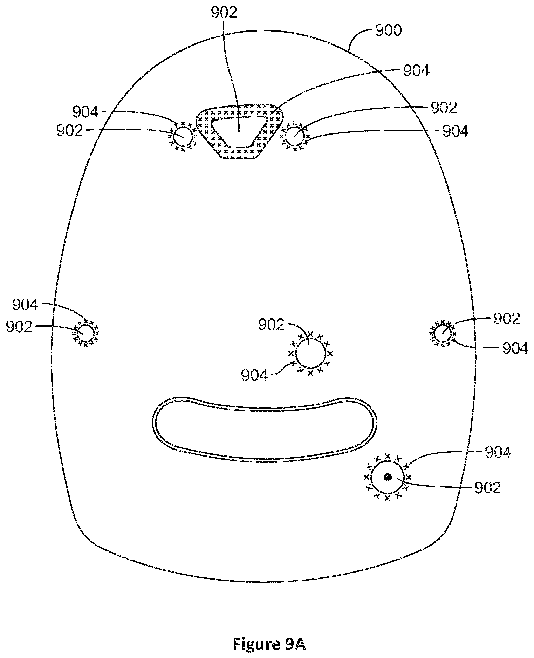

It may be desirable to put stronger adhesive around holes or other structures that protrude through the adhesive layer to prevent peeling of the adhesive layer at edges that surround such holes or structures. Figure 9A depicts the adhesive layer 900 with examples of holes and structures 902 around which stronger adhesive may be applied. These holes or structures 902 may be areas where an air valve, a fill hole, a needle or cannula hole, a patient interface hole, a speaker opening, a light or LED opening, a reset button, a kill switch, or other element is located and necessitates a hole or space or break in the adhesive layer. These holes or structures 902 may also represent areas where a manufacturing operation necessitates holes, for example, to hold the adhesive layer 900 during a step in the manufacturing process. In an exemplary embodiment, it may be desirable to form a stronger adhesive 904 around all or most of the holes and/or structures 902 in the adhesive layer 900 so as to better secure the adhesive layer 900 to the skin at these locations. Because the adhesive layer 900 typically begins to pull apart from the skin surface at its edges, including potentially at edges of holes in the adhesive layer 900, using a stronger adhesive at these locations would enhance the wear time of the adhesive and medical device to the user's skin, while not substantially increasing the discomfort on the user's skin as may happen if the entire surface of the adhesive layer 900 were formed with a stronger adhesive. Examples of a "stronger" adhesive are described above in terms of % of peel strength and/or wear time. A less strong adhesive may be used elsewhere for the adhesive layer as indicated by the cross hatching on the adhesive layer 900 where the stronger adhesive is not applied in Figure 9A.

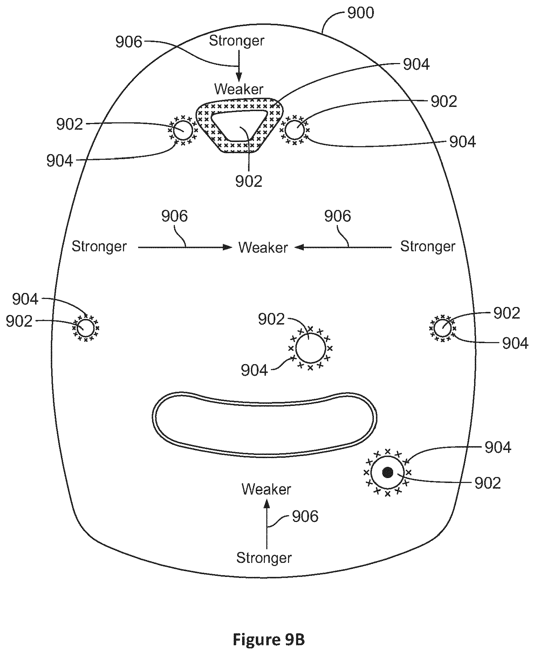

In another exemplary embodiment, the adhesive may form a gradient of strength from an outer perimeter of the adhesive layer 900 where the adhesive may be stronger, and gradually weaken moving toward the center of the adhesive layer 900 where the adhesive typically doesn't need to be as strong since the interior portions may not experience the same stresses as the perimeter of the adhesive layer 900. An example of such a gradient of strength on the adhesive layer 900 is shown in Figure 9B. The stronger adhesive at the outer perimeter may be 10% to 100% stronger than the weaker adhesive at the center of the adhesive layer 900, or more particularly, 10% to 50%; or in an exemplary embodiment, 20% stronger. The adhesive may gradually weaken at positions on the adhesive layer 900 located radially inward from the perimeter until it becomes what has been referred to above as the "weaker adhesive." Arrows 906 indicate the gradient from stronger adhesive on the perimeter to weaker adhesive in the radial inner positions that are closer to the center of the adhesive layer 900. This gradient may be created by applying different amounts or different types of adhesive across the surface of the adhesive layer 900, such that the perimeter has a greater amount or (stronger) type of adhesive material, in particular a greater amount of a stronger type of adhesive, than the interior or near the center of the adhesive layer 900, which may result in a stronger adhesive property.

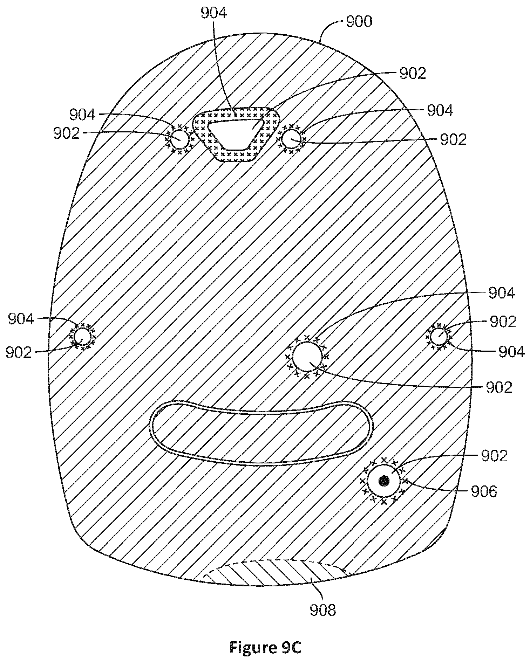

In a further exemplary embodiment as depicted in Figure 9C, a portion of the perimeter of the adhesive layer 900 may have a weaker adhesive then other portions of the perimeter or than other portions of the adhesive layer 900. For example, one portion (such as a portion 908 of the bottom end) of the adhesive layer 900 may have a weaker adhesive (or in some embodiments, no adhesive) thereby making it easier to separate from a user's skin, so as to allow the user to insert their finger at that portion 908 under the adhesive layer and above the skin when it comes time to remove the adhesive layer 900 from the skin (e.g., at the end of life of the on-body medical device). This portion 908 may be arcuate in shape or in the shape of a fingertip. By allowing a user to insert their fingertip at this weaker adhesive portion 908 when it comes time to remove the adhesive layer 900 from the skin, it may be easier for the user to start the adhesive removal process of the rest of the adhesive layer 900. Once the user is able to separate or lift this portion of the adhesive layer 900, the user can grab this separated portion and gradually lift the adhesive layer 900 in a peeling fashion to remove the remainder of the adhesive layer 900, where the remainder of the adhesive layer 900 has a stronger adhesive than this particular portion of the perimeter.

While exemplary embodiments have been described herein, various changes in form and detail may be made without departing from the intended scope of the appended claims.

## Claims

1. An on-body medical device (100), comprising:
a housing (102) having a skin-facing surface configured for facing the skin (106) of a user; and
an adhesive layer (701) secured to the skin-facing surface of the housing for securing the on-body medical device to the skin of the user, **characterized by** islands (704) of adhesive on the adhesive layer, and
a different strength adhesive applied around a perimeter (702) of the adhesive layer.

2. The on-body medical device (100) of claim 1, wherein the islands (704) are periodically spaced apart and/or wherein the islands are arranged in a grid.

3. The on-body medical device (100) of claim 1 or 2, wherein the islands (704) have a weaker adhesive than the adhesive applied around the perimeter of the adhesive layer (701) and the weaker adhesive is a 1 day wear time or a 2 day wear time adhesive.

4. The on-body medical device (100) of any one of claims 2 to 3, wherein the stronger adhesive is a 3 day wear term or a 4 day wear term adhesive.

5. The on-body medical device (100) of any one of claims 1 to 4, wherein there is no adhesive present under the islands (704).

6. The on-body medical device (100) of any one of claims 1 to 4, wherein there is an adhesive present under the islands (704).

7. The on-body medical device (100) of claim 6, wherein a stronger adhesive than that of the islands (704) is present under the islands.

8. The on-body medical device (100) of any one of claims 1-7, wherein the different strength adhesive is stronger than the adhesive of the islands (704), wherein the different strength adhesive also is applied around a region interior to the perimeter so as to surround the islands, and wherein the different strength adhesive is at least 20% stronger than the adhesive of the islands.

9. The-on-body medical device (100) of claim 1, wherein the islands (704) are a stronger strength adhesive,
wherein a weaker strength adhesive is applied around a perimeter of the adhesive layer (701) and also applied around a region interior to the perimeter so as to surround the islands with the weaker strength adhesive, and wherein the stronger strength adhesive is at least 20% stronger than the weaker strength adhesive.

10. The on-body medical device (100) of claim 1, further comprising:
a needle and/or cannula insertion assembly for inserting a needle and/or cannula (306) through the skin (106) of the user to deliver a medicament to the user;
an opening in the skin-facing surface; and
a corresponding opening (304) in the adhesive layer for enabling the needle and/or the cannula to be inserted through the skin of the user.

11. A method, comprising:
creating islands (704) of a first adhesive on an adhesive layer (701) applying an adhesive of a second strength that differs from the first strength around the perimeter of the adhesive layer; and
securing the adhesive layer to a skin-facing surface of a housing (102) of an on-body medical device (100).

12. The method of claim 11, wherein the islands (704) are periodically spaced apart and/or wherein the islands are arranged in a grid.

13. The method of claims 11 or 12, wherein the islands (704) have a weaker adhesive than the adhesive applied around the perimeter of the adhesive layer and the weaker adhesive is a 1 day wear time or a 2 day wear time adhesive.

14. The method of claim 13, wherein the second adhesive is a 3 day wear term or a 4 day wear term adhesive.

15. The method of one of claims 11-14, wherein there is no adhesive present under the islands (704).

16. The method of claims 11-14, further comprising applying an adhesive to the adhesive layer that is under the islands (704).

17. The method of claim 16, wherein a stronger adhesive than that of the islands is applied to be under the islands (704).

18. The method of claims 11-17, wherein the different strength adhesive is stronger than the adhesive of the islands (704), wherein the method further comprises also applying the different strength adhesive around a region interior to the perimeter so as to surround the islands, and wherein the different strength adhesive is at least 20% stronger than the adhesive of the islands.

19. The method of claim 11, wherein the islands (704) are a stronger strength adhesive, wherein a weaker strength adhesive is applied around a perimeter of the adhesive layer and is also applied around a region interior to the perimeter so as to surround the islands with the weaker strength adhesive, and wherein the stronger strength adhesive is at least 20% stronger than the weaker strength adhesive.

20. The method of claim 11, wherein the on-body medical device (100) includes a needle and/or cannula insertion assembly for inserting a needle and/or cannula (306) through the skin (106) of the user to deliver a medicament to the user, wherein the housing (102) includes an opening in the skin-facing surface, and wherein the method further comprises providing a corresponding opening (304) in the adhesive layer for enabling the needle and/or the cannula to be inserted through the skin of the user.

## Patentansprüche

1. Am Körper tragbare medizinische Vorrichtung (100), die Folgendes umfasst:
ein Gehäuse (102) mit einer hautzugewandten Oberfläche, die dazu ausgelegt ist, der Haut (106) eines Benutzers zugewandt zu sein; und
eine Haftschicht (701), die an der hautzugewandten Oberfläche des Gehäuses befestigt ist, zur Befestigung der am Körper tragbaren medizinische Vorrichtung an der Haut des Benutzers, **gekennzeichnet durch** Klebstoffinseln (704) auf der Haftschicht, und
einen Klebstoff unterschiedlicher Festigkeit, der um einen Umfang (702) der Haftschicht herum aufgebracht ist.

2. Am Körper tragbare medizinische Vorrichtung (100) nach Anspruch 1, wobei die Inseln (704) periodisch voneinander beabstandet sind und/oder wobei die Inseln in einem Gittermuster angeordnet sind.

3. Am Körper tragbare medizinische Vorrichtung (100) nach Anspruch 1 oder 2, wobei die Inseln (704) einen schwächeren Klebstoff aufweisen als der Klebstoff, der um den Umfang der Haftschicht (701) herum aufgebracht ist, und der schwächere Klebstoff ein 1-Tage-Tragezeit- oder ein 2-Tage-Tragezeit-Klebstoff ist.

4. Am Körper tragbare medizinische Vorrichtung (100) nach einem der Ansprüche 2 bis 3, wobei der stärkere Klebstoff ein 3-Tage-Tragezeit- oder ein 4-Tage-Tragezeit-Klebstoff ist.

5. Am Körper tragbare medizinische Vorrichtung (100) nach einem der Ansprüche 1 bis 4, wobei kein Klebstoff unter den Inseln (704) vorhanden ist.

6. Am Körper tragbare medizinische Vorrichtung (100) nach einem der Ansprüche 1 bis 4, wobei ein Klebstoff unter den Inseln (704) vorhanden ist.

7. Am Körper tragbare medizinische Vorrichtung (100) nach Anspruch 6, wobei ein stärkerer Klebstoff als der der Inseln (704) unter den Inseln vorhanden ist.

8. Am Körper tragbare medizinische Vorrichtung (100) nach einem der Ansprüche 1-7, wobei der Klebstoff unterschiedlicher Festigkeit stärker ist als der Klebstoff der Inseln (704), wobei der Klebstoff unterschiedlicher Festigkeit auch um einen Bereich innerhalb des Umfangs herum aufgebracht ist, um die Inseln zu umgeben, und wobei der Klebstoff unterschiedlicher Festigkeit mindestens 20 % stärker ist als der Klebstoff der Inseln.

9. Am Körper tragbare medizinische Vorrichtung (100) nach Anspruch 1, wobei es sich bei den Inseln (704) um einen Klebstoff stärkerer Festigkeit handelt,
wobei ein Klebstoff schwächerer Festigkeit um einen Umfang der Haftschicht (701) herum aufgebracht ist und auch um einen Bereich innerhalb des Umfangs herum aufgebracht ist, um die Inseln mit dem Klebstoff schwächerer Festigkeit zu umgeben, und wobei der Klebstoff stärkerer Festigkeit mindestens 20 % stärker als der Klebstoff schwächerer Festigkeit ist.

10. Am Körper tragbare medizinische Vorrichtung (100) nach Anspruch 1, die ferner Folgendes umfasst:
eine Nadel- und/oder Kanülen-Einführungsanordnung zum Einführen einer Nadel und/oder Kanüle (306) durch die Haut (106) des Benutzers, um ein Medikament an den Benutzer abzugeben;
eine Öffnung in der hautzugewandten Oberfläche; und
eine entsprechende Öffnung (304) in der Haftschicht, um das Einführen der Nadel und/oder der Kanüle durch die Haut des Benutzers zu ermöglichen.

11. Verfahren, umfassend:
Erzeugen von Inseln (704) eines ersten Klebstoffs auf einer Haftschicht (701), Aufbringen eines Klebstoff einer zweiten Festigkeit, die sich von der ersten Festigkeit unterscheidet, um den Umfang der Haftschicht herum; und
Befestigen der Haftschicht an einer hautzugewandten Oberfläche eines Gehäuses (102) einer am Körper tragbaren medizinischen Vorrichtung (100).

12. Verfahren nach Anspruch 11, wobei die Inseln (704) periodisch voneinander beabstandet sind und/oder wobei die Inseln in einem Gittermuster angeordnet sind.

13. Verfahren nach Anspruch 11 oder 12, wobei die Inseln (704) einen schwächeren Klebstoff aufweisen als der Klebstoff, der um den Umfang der Haftschicht aufgebracht ist, und der schwächere Klebstoff ein 1-Tage-Tragezeit- oder ein 2-Tage-Tragezeit-Klebstoff ist.

14. Verfahren nach Anspruch 13, wobei der zweite Klebstoff ein 3-Tage-Tragezeit- oder ein 4-Tage-Tragezeit-Klebstoff ist.

15. Verfahren nach einem der Ansprüche 11-14, wobei kein Klebstoff unter den Inseln (704) vorhanden ist.

16. Verfahren nach den Ansprüchen 11-14, ferner umfassend das Aufbringen eines Klebstoffs auf die Haftschicht, die sich unter den Inseln (704) befindet.

17. Verfahren nach Anspruch 16, wobei ein stärkerer Klebstoff als der der Inseln so aufgebracht wird, dass er sich unter den Inseln befindet (704).

18. Verfahren nach den Ansprüchen 11-17, wobei der Klebstoff unterschiedlicher Festigkeit stärker als der Klebstoff der Inseln (704) ist, wobei das Verfahren ferner das Aufbringen des Klebstoffs unterschiedlicher Festigkeit um einen Bereich innerhalb des Umfangs herum umfasst, um die Inseln zu umgeben, und wobei der Klebstoff unterschiedlicher Festigkeit mindestens 20 % stärker als der Klebstoff der Inseln ist.

19. Verfahren nach Anspruch 11, wobei die Inseln (704) ein Klebstoff stärkerer Festigkeit sind, wobei ein Klebstoff schwächerer Festigkeit um einen Umfang der Haftschicht herum aufgebracht wird und auch um einen Bereich innerhalb des Umfangs herum aufgebracht wird, um die Inseln mit dem Klebstoff schwächerer Festigkeit zu umgeben, und wobei der Klebstoff stärkerer Festigkeit mindestens 20 % stärker als der Klebstoff schwächerer Festigkeit ist.

20. Verfahren nach Anspruch 11, wobei die am Körper tragbare medizinische Vorrichtung (100) eine Nadel- und/oder Kanülen-Einführungsanordnung zum Einführen einer Nadel und/oder Kanüle (306) durch die Haut (106) des Benutzers beinhaltet, um ein Medikament an den Benutzer abzugeben, wobei das Gehäuse (102) eine Öffnung in der hautzugewandten Oberfläche beinhaltet, und wobei das Verfahren ferner das Bereitstellen einer entsprechenden Öffnung (304) in der Haftschicht umfasst, um das Einführen der Nadel und/oder der Kanüle durch die Haut des Benutzers zu ermöglichen.

## Revendications

1. Dispositif médical sur le corps (100), comprenant :
un boîtier (102) ayant une surface faisant face à la peau configuré pour faire face à la peau (106) d'un utilisateur ; et
une couche d'adhésif (701) fixée sur la surface faisant face à la peau du boîtier pour fixer le dispositif médical sur le corps sur la peau de l'utilisateur, **caractérisée par** des îlots (704) d'adhésif sur la couche d'adhésif, et
un adhésif de résistance différente appliqué autour d'un périmètre (702) de la couche d'adhésif.

2. Dispositif médical sur le corps (100) selon la revendication 1, dans lequel les îlots (704) sont espacés périodiquement et/ou dans lequel les îlots sont agencés en une grille.

3. Dispositif médical sur le corps (100) selon la revendication 1 ou 2, dans lequel les îlots (704) ont un adhésif plus faible que l'adhésif appliqué autour du périmètre de la couche d'adhésif (701) et l'adhésif plus faible est un adhésif à durée d'usure de 1 jour ou un adhésif à durée d'usure de 2 jours.

4. Dispositif médical sur le corps (100) selon l'une quelconque des revendications 2 à 3, dans lequel l'adhésif plus fort est un adhésif à durée d'usure de 3 jours ou un adhésif à durée d'usure de 4 jours.

5. Dispositif médical sur le corps (100) selon l'une quelconque des revendications 1 à 4, dans lequel aucun adhésif n'est présent sous les îlots (704).

6. Dispositif médical sur le corps (100) selon l'une quelconque des revendications 1 à 4, dans lequel un adhésif est présent sous les îlots (704).

7. Dispositif médical sur le corps (100) selon la revendication 6, dans lequel un adhésif plus fort que celui des îlots (704) est présent sous les îlots.

8. Dispositif médical sur le corps (100) selon l'une quelconque des revendications 1 à 7, dans lequel l'adhésif de résistance différente est plus fort que l'adhésif des îlots (704), dans lequel l'adhésif de résistance différente est également appliqué autour d'une zone intérieure au périmètre de manière à entourer les îlots, et dans lequel l'adhésif de résistance différente est au moins 20 % plus résistant que l'adhésif des îlots.

9. Dispositif médical sur le corps (100) selon la revendication 1, dans lequel les îlots (704) sont un adhésif à résistance plus forte,
dans lequel un adhésif à résistance plus faible est appliqué autour d'un périmètre de la couche d'adhésif (701) et également appliqué autour d'une zone intérieure au périmètre de manière à entourer les îlots de l'adhésif à résistance plus faible, et dans lequel l'adhésif à résistance plus fort est au moins 20 % plus résistant que l'adhésif à résistance plus faible.

10. Dispositif médical sur le corps (100) selon la revendication 1, comprenant en outre :
un ensemble d'insertion d'aiguille et/ou de canule pour insérer une aiguille et/ou une canule (306) à travers la peau (106) de l'utilisateur afin d'administrer un médicament à l'utilisateur ;
une ouverture dans la surface faisant face à la peau ; et
une ouverture correspondante (304) dans la couche d'adhésif pour permettre l'insertion de l'aiguille et/ou la canule à travers la peau de l'utilisateur.

11. Procédé, comprenant :
la création d'îlots (704) d'un premier adhésif sur une couche d'adhésif (701) appliquant un adhésif d'une deuxième résistance différente de la première résistance sur le périmètre de la couche d'adhésif ; et
la fixation de la couche d'adhésif sur une surface faisant face à la peau d'un boîtier (102) d'un dispositif médical sur le corps (100).

12. Procédé selon la revendication 11, dans lequel les îlots (704) sont espacés périodiquement et/ou dans lequel les îlots sont agencés en une grille.

13. Procédé selon la revendication 11 ou 12, dans lequel les îlots (704) ont un adhésif plus faible que l'adhésif appliqué autour du périmètre de la couche d'adhésif et l'adhésif plus faible est un adhésif à durée d'usure de 1 jour ou à durée d'usure de 2 jours.

14. Procédé selon la revendication 13, dans lequel le second adhésif est un adhésif à durée d'usure de 3 jours ou un adhésif à durée d'usure de 4 jours.

15. Procédé selon l'une des revendications 11 à 14, dans lequel il n'y a pas d'adhésif présent sous les îlots (704).

16. Procédé selon les revendications 11 à 14, comprenant en outre l'application d'un adhésif sur la couche d'adhésif qui se trouve sous les îlots (704).

17. Procédé selon la revendication 16, dans lequel un adhésif plus fort que celui des îlots est appliqué pour être sous les îlots (704).

18. Procédé selon les revendications 11 à 17, dans lequel l'adhésif de résistance différente est plus fort que l'adhésif des îlots (704), dans lequel le procédé comprend en outre l'application également de l'adhésif de résistance différente autour d'une zone intérieure au périmètre de manière à entourer les îlots, et dans lequel l'adhésif de résistance différente est au moins 20 % plus fort que l'adhésif des îlots.

19. Procédé selon la revendication 11, dans lequel les îlots (704) sont un adhésif à résistance plus forte, dans lequel un adhésif à résistance plus faible est appliqué autour d'un périmètre de la couche d'adhésif et est également appliqué autour d'une zone intérieure au périmètre de manière à entourer les îlots avec l'adhésif de résistance plus faible, et dans lequel l'adhésif à résistance plus forte est au moins 20 % plus résistant que l'adhésif à résistance plus faible.

20. Procédé selon la revendication 11, dans lequel le dispositif médical sur le corps (100) comprend un ensemble d'insertion d'aiguille et/ou de canule pour insérer une aiguille et/ou une canule (306) à travers la peau (106) de l'utilisateur pour administrer un médicament à l'utilisateur, dans lequel le boîtier (102) comprend une ouverture dans la surface faisant face à la peau, et dans lequel le procédé comprend en outre la fourniture d'une ouverture correspondante (304) dans la couche d'adhésif pour permettre à l'aiguille et/ou à la canule d'être insérée(s) à travers la peau de l'utilisateur.
